# EUROPÄISCHE PATENTSCHRIFT

(11) **EP 0 657 181 B1**
(45) Veröffentlichungstag und Bekanntmachung des Hinweises auf die Patenterteilung: **17.03.1999**
(21) Anmeldenummer: 94115342.1
(22) Anmeldetag: 29.09.1994
(51) Int. Cl.: A61M 16/00

(54) **Beatmungsbeutel**
Artificial respiration bag
Ballon d'insufflateur manuel

(30) Priorität: 08.12.1993 DE 4341746
(43) Veröffentlichungstag der Anmeldung: 14.06.1995
(73) Patentinhaber: Heraeus Med GmbH, D-63450 Hanau (DE)
(72) Erfinder: Kühn, Hans-Joachim, D-65197 Wiesbaden (DE)
(74) Vertreter: Kühn, Hans-Christian

(56) Entgegenhaltungen:
- EP-A- 0 139 363
- DE-A- 1 616 421
- FR-A- 1 183 803
- US-A- 3 046 978
- US-A- 3 262 446
- US-A- 3 548 822

## Beschreibung

Die Erfindung betrifft einen Beatmungsbeutel für ein handbetätigbares Beatmungsgerät zur künstlichen Beatmung von Menschen, der aus einem elastischen, luftabschließendem Material in Form eines langgestreckten Hohlkörpers gebildet ist, mit einem senkrecht zur Längsachse im wesentlichen kreisförmigen Querschnitt und mit Griffflächen zur differenzierten Reduzierung des Volumens des Hohlkörpers, und der eine Lufteintrittsöffnung und eine Luftaustrittsöffnung aufweist, wobei die Griffflächen im wesentlichen konzentrisch und in Richtung der Längsachse hintereinander an mindestens einem Ende des Hohlkörpers um die Längsachse herum angeordnet sind und wobei der Durchmesser des Hohlkörpers zu dem mindestens einen, Griffflächen aufweisenden Ende hin abnimmt.

Ein derartiger Beatmungsbeutel ist aus DE-GM 18 72 078 bekannt. Hier ist ein Beatmungsbeutel beschrieben, an dessen Umfang Rinnen zur Aufnahme von Fingern angeordnet sind. Durch diese Rinnen ist die Anordnung der Finger streng fixiert, eine andere Fingeranordnung ist bei diesem Beatmungsbeutel kaum möglich. Dieser Beatmungsbeutel ist optimal für eine festgelegte Handgröße ausgelegt; für davon aubweichende Handgrößen dürfte dieser Beatmungsbeutel nur erschwert bedienbar sein. Die Rinnen für die Finger sind dabei strahlenförmig gegenüber der Rinne für den Daumen angeordnet und bilden die Griffflächen des Beatmungsbeutels. Eine differenzierte Reduzierung des Volumens des Hohlkörpers ist hiermit nur sehr eingeschränkt möglich, da eine solche Differenzierung bestenfalls durch mehr oder weniger starkes Zusammendrücken durch die Bedienperson erfolgen kann, wobei die Abstufung des Volumens stets unter dem subjektiven Eindruck der Bedienperson erfolgt, der dabei keinerlei objektives Beurteilungskriterium zur Verfügung steht. Gerade eine objektive Beurteilung des applizierten Luftvolumens ist jedoch notwendig, um eine Schädigung der zu beatmenden Person auszuschließen.

Ein weiterer Beatmungsbeutel ist beispielsweise aus EP 0 091 428 bekannt. Hier ist ein Beatmungsbeutel aus einem elastischen und vorzugsweise langgestreckten Hohlkörper mit im wesentlichen kreisförmigen Querschnitt beschrieben, der an seinen Längsenden jeweils eine Öffnung aufweist, an der ein Lufteinlaß- bzw. Luftauslaßstutzen angeschlossen werden kann. Der Hohlkörper weist in seiner Wandung zwei sich in axialer Richtung erstreckende Einbuchtungen auf, die in einem Winkel von etwa 100 bis 140°, bezogen auf den Querschnitt des Hohlkörpers, angeordnet sind. Im Querschnitt gesehen ergeben sich dadurch zwei zwischen den Einbuchtungen gelegene Beutelabschnitte, von denen der eine größer als der andere ist. Eine Person, die den Beatmungsbeutel bedient, kann nun wählen, ob sie die beiden Einbuchtungen von dem kleineren Beutelabschnitt her oder von dem größeren Beutelabschnitt her umfaßt. Auf diese Weise kann das Beutelvolumen beim Zusammendrücken mehr oder weniger stark reduziert werden. Das dem zu beatmenden Menschen über den Beatmungsbeutel zugeführte Gasvolumen ist also davon abhängig, von welcher Seite der Beatmungsbeutel erfaßt wird. Dabei wird zur Beatmung beispielsweise eines Kindes die kleinere Volumenverringerung gewählt, während zur Beatmung einer erwachsenen Person der Beutel so erfaßt wird, daß eine größere Volumenreduzierung beim Zusammenpressen erfolgt. Auf diese Weise ist es möglich, die unterschiedlichen Bedürfnisse unterschiedlich großer Menschen bei der künstlichen Beatmung zu berücksichtigen. Allerdings ist die Volumenreduzierung auf diese Weise mehr oder weniger ungenau, da stets nur ein kleiner Teil der Länge des Beutels von der Hand der Bedienperson erfaßt wird, so daß das darüber hinausgehende Volumen lediglich durch die Eigenelastizität des Beatmungsbeutels mehr oder weniger stark reduziert wird. Zudem ist die Anordnung der als Griffflächen dienenden Einbuchtungen ergonomisch ungünstig, so daß bei der Bedienperson nach längerer Beatmung mit dem Beatmungsbeutel leicht Ermüdungserscheinungen auftreten können.

Aus DE-A-1 616 421 ist ein Beatmungsbeutel bekannt, der in Richtung seiner Längsachse zusammenlegbar ist, um platzsparend verpackt werden zu können. Dieser Beutel weist eine etwa zentral gelegene, zylindrische Grifffläche auf. Der Durchmesser des Beutels nimmt zu den beiden Enden hin stufenweise ab, wobei diese ringförmigen, elastischen Stufen gegenüber dem zylindrischen Wandabschnitt eine vergrößere Oberfläche aufweisen und über Zonen mit sehr dünner Wandstärke miteinander verbunden sind, um ein Zusammenlegen zu ermöglichen. US-A-3 046 978 offenbart einen Beatmungsbeutel, mit dem unterschiedliche Atemvolumina appliziert werden können. Die unterschiedlichen Volumina werden gewährleistet durch innerhalb des Beutels angeordnete Kugeln unterschiedlichen Durchmessers in Zusammenspiel mit einer doppelten Beutelwand, in die Luft einströmen kann.

Ausgehend von dem vorstehend genannten Stand der Technik stellt sich die Erfindung die Aufgabe, einen Beatmungsbeutel zu schaffen, mit dem unterschiedliche Atemvolumina mit relativ hoher Genauigkeit appliziert werden können, wobei die Ermüdungserscheinungen der den Beatmungsbeutel bedienenden Person auf ein Minimum reduziert werden.

Die Aufgabe wird für einen Beatmungsbeutel gemäß dem Oberbegriff des Anspruchs 1 erfindungsgemäß dadurch gelöst, daß der Durchmesser des Hohlkörpers stufenförmig abnimmt, wobei jede Grifffläche jeweils eine Stufe bildet und daß die jeweils zwischen zwei Griffflächen gebildeten Bereiche mit konkaver Krümmung eine gegenüber der durchschnittlichen Wandstärke des Hohlkörpers vergrößerte Wandstärke aufweist. Bei einem derart gestalteten Beatmungsbeutel erfolgt eine im Volumen differenzierte Beatmung dadurch, daß der Beutel je nach zu applizierendem Gasvolumen entweder an seinem größten Umfang oder an einer der Griffflächen, die an dem Teil des Hohlkörpers mit verringertem Durchmesser angeordnet sind, zusammengedruckt wird. Dabei kann jeder der Griffflächen ein bestimmtes Gasvolumen zugeordnet werden, so daß entsprechend der Anzahl der Griffflächen eine Anzahl verschiedener Luftvolumina zur Verfügung steht. Die Anordnung der Griffflächen zur volumenreduzierten Luftapplikation an dem in seinem Durchmesser reduzierten Ende des Hohlkörpers ist zudem ergonomisch günstiger, so daß Verspannungen in der Hand bzw. im Arm der Bedienperson weitgehend vermieden werden.

Vorteilhaft weist der Beatmungsbeutel genau zwei den Durchmesser des Hohlkörpers verringernde Stufen auf, so daß insgesamt drei verschiedene Atemvolumina appliziert werden können. Dies ist eine Anzahl, die den praktischen Bedürfnissen weitestgehend gerecht wird.

Vorteilhaft für eine relativ einfache und kostengünstig herstellbare Beutelform ist es, daß genau ein Ende des Hohlkörpers Griffflächen aufweist.

Die Wirkungsweise des Beatmungsbeutels wird sowohl hinsichtlich der Genauigkeit des zu applizierenden Atemvolumens als auch hinsichtlich der Selbstexpansion des Beatmungsbeutels nach dem Zusammendrücken dadurch vorteilhaft beeinflußt, daß die Vergrößerung der Wandstärke in den Bereichen mit konkaver Krümmung mindestens 25 %, insbesondere etwa 50 % bis 100 % beträgt.

Eine Erleichterung für die Bedienperson besteht darin, daß die Griffflächen durch Erhebungen an der Oberfläche des Hohlkörpers markiert sein können.

Nachstehend wird die Erfindung anhand einer Zeichnung beispielhaft näher erläutert. In der Zeichnung zeigt
- Figur 1: eine Schnittdarstellung eines Beatmungsbeutels und
- Figur 2: die Seitenansicht eines Beatmungsbeutels.

Der Beatmungsbeutel weist einen Hohlkörper 1 aus weitestgehend luftdichtem Material, z. B. Silikongummi auf. Der Hohlkörper 1 besitzt eine in seinem Hauptteil 2 zylindrische Form, der Durchmesser des Hohlkörpers 1 nimmt zu einem Ende hin stufenförmig in zwei Stufen ab, die die Griffflächen 3 bilden. An dem Ende mit dem größeren Durchmesser weist der Hohlkörper 1 eine Lufteinlaßöffnung 4 auf und an seinem stufenförmig im Durchmesser verringerten Ende ist eine Luftauslaßöffnung 5 angeordnet, durch die Atemluft durch ein Ventil zum Patienten geführt wird. Die jeweils zwischen zwei Griffflächen 3 gebildeten Bereiche mit konkaver Krümmung 6 weisen eine gegenüber der durchschnittlichen Wandstärke des Hohlkörpers 1 vergrößerte Wandstärke auf. Die Vergrößerung der Wandstärke beträgt etwa 75 %. Zusätzlich sind an der Innenseite des Hohlkörpers 1 im Bereich seines zylinderförmigen Hauptteils 2 ringförmige Verstärkungen 7 der Wand angeordnet, die auch bei Zusammendrücken des Hauptteils 2 eine zuverlässige Selbstexpansion des Beatmungsbeutels sicherstellen. Die Vergrößerung der Wandstärke des Hohlkörpers 1 im Bereich dieser ringförmigen Verstärkung 7 beträgt ebenfalls etwa 75 %.

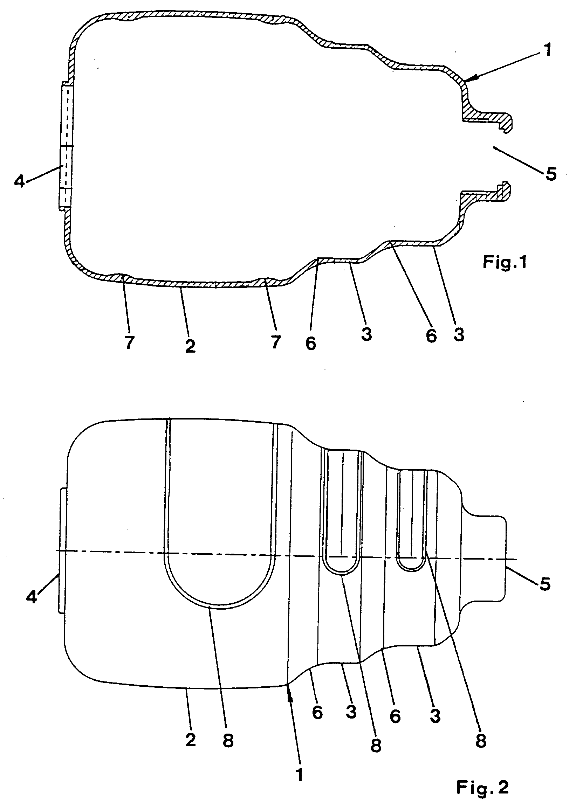

In Figur 2 sind die Griffflächen 3 zusätzlich durch Erhebungen 8 markiert, um dem Bedienpersonal eine eindeutige optisch oder auch durch Tasten wahrnehmbare Hilfestellung zu geben.

## Patentansprüche

1. Beatmungsbeutel für ein handbetätigbares Beatmungsgerät zur künstlichen Beatmung von Menschen, der aus einem elastischen, luftabschließenden Material in Form eines langgestreckten Hohlkörpers mit einer durchschnittlichen Wandstärke gebildet ist, mit einem senkrecht zur Längsachse im wesentlichen kreisförmigen Querschnitt und mit Griffflächen zur differenzierten Reduzierung des Volumens des Hohlkörpers, der eine Lufteintrittsöffnung und eine Luftaustrittsöffnung aufweist, wobei die Griffflächen (3) im wesentlichen konzentrisch und in Richtung der Längsachse hintereinander an mindestens einem Ende des Hohlkörpers (1) um die Längsachse herum angeordnet sind und wobei der Durchmesser des Hohlkörpers (1) zu dem mindestens einen, Griffflächen (3) aufweisenden Ende hin abnimmt, dadurch gekennzeichnet, daß der Durchmesser des Hohlkörpers (1) stufenförmig abnimmt, wobei jede Grifffläche (3) jeweils eine Stufe bildet und daß die jeweils zwischen zwei Griffflächen (3) gebildeten Bereiche mit konkaver Krümmung (6) eine gegenüber der durchschnittlichen Wandstärke des Hohlkörpers (1) vergrößerte Wandstärke aufweisen.

2. Beatmungsbeutel nach Anspruch 1, dadurch gekennzeichnet, daß der Durchmesser des Hohlkörpers (1) durch genau zwei Stufen verringert ist.

3. Beatmungsbeutel nach Anspruch 1 oder 2, dadurch gekennzeichnet, daß genau ein Ende des Hohlkörpers (1) Griffflächen (3) aufweist.

4. Beatmungsbeutel nach einem der Ansprüche 1 bis 3, dadurch gekennzeichnet, daß die Vergrößerung der Wandstärke in den Bereichen mit konkaver Krümmung (6) mindestens 25% beträgt.

5. Beatmungsbeutel nach Anspruch 4, dadurch gekennzeichnet, daß die Vergrößerung der Wandstärke etwa 50% bis 100% beträgt.

6. Beatmungsbeutel nach einem der Ansprüche 1 bis 5, dadurch gekennzeichnet, daß die Griffflächen (3) durch Erhebungen (8) an der Oberfläche des Hohlkörpers (1) markiert sind.

## Claims

1. A respiration bag for a hand-operable respiration apparatus for the artificial respiration of persons, which is formed from an elastic, air-excluding material in the form of an elongated hollow body with an average wall thickness, with a substantially circular cross-section perpendicular to the longitudinal axis and with grip faces for the differentiated reduction of the volume of the hollow body, which has an air inlet opening and an air outlet opening, the grip faces (3) being arranged substantially concentrically and one behind the other in the direction of the longitudinal axis at at least one end of the hollow body (1) around the longitudinal axis and the diameter of the hollow body (1) decreasing towards the at least one end having grip faces (3), characterised in that the diameter of the hollow body (1) decreases by stages, each grip face (3) respectively forming a stage and that the regions with a concave curvature (6) respectively formed between two grip faces (3) have an increased wall thickness compared with the average wall thickness of the hollow body (1).

2. A respiration bag according to Claim 1, characterised in that the diameter of the hollow body (1) is reduced by precisely two stages.

3. A respiration bag according to Claim 1 or 2, characterised in that precisely one end of the hollow body (1) has grip faces (3).

4. A respiration bag according to one of Claims 1 to 3, characterised in that the increase to the wall thickness in the regions with concave curvature (6) amounts to at least 25 %.

5. A respiration bag according to Claim 4, characterised in that the increase to the wall thickness amounts to approximately 50 % to 100 %.

6. A respiration bag according to one of Claims 1 to 5, characterised in that the grip faces (3) are marked by elevations (8) on the surface of the hollow body (1).

## Revendications

1. Ballon insufflateur pour un insufflateur à actionnement manuel destiné à l'insufflation humaine artificielle, qui est réalisé en matériau élastique imperméable à l'air sous forme d'un corps creux allongé avec une épaisseur de paroi moyenne, avec une section transversale sensiblement circulaire perpendiculairement à l'axe longitudinal, et avec des surfaces de préhension pour la réduction différenciée du volume du corps creux, et qui présente une ouverture d'entrée d'air et une ouverture de sortie d'air, les surfaces de préhension (3) étant agencées sensiblement concentriquement autour de l'axe longitudinal et les unes derrière les autres en direction de l'axe longitudinal à une extrémité au moins du corps creux (1), et le diamètre du corps creux (1) diminuant vers ladite au moins une extrémité comprenant les surfaces de préhension (3), caractérisé en ce que le diamètre du corps creux (1) diminue en gradins, chaque surface de préhension (3) formant un gradin respectif, et en ce que les zones à courbure concave (6) formées entre deux surfaces de préhension respectives (3) présentent une épaisseur de paroi augmentée par rapport a l'épaisseur de paroi moyenne du corps creux (1).

2. Ballon insufflateur selon la revendication 1, caractérisé en ce que le diamètre du corps creux (1) est réduit exactement par deux gradins.

3. Ballon insufflateur selon l'une ou l'autre des revendications 1 et 2, caractérisé en ce qu'une extrémité du corps creux (1) exactement présente des surfaces de préhension (3).

4. Ballon insufflateur selon l'une quelconque des revendications 1 à 3, caractérisé en ce que l'augmentation de l'épaisseur de paroi dans les zones à courbure concave (6) s'élève à au moins 25 %.

5. Ballon insufflateur selon la revendication 4, caractérisé en ce que l'augmentation de l'épaisseur de paroi est d'environ 50 % à 100 %.

6. Ballon insufflateur selon l'une quelconque des revendications 1 à 5, caractérisé en ce que les surfaces de préhension (3) sont marquées par des surélévations (8) à la surface du corps creux (1).
